# EUROPÄISCHE PATENTSCHRIFT

(11) **EP 1 576 936 B1**
(45) Veröffentlichungstag und Bekanntmachung des Hinweises auf die Patenterteilung: **19.12.2007**
(21) Anmeldenummer: 05002834.9
(22) Anmeldetag: 10.02.2005
(51) Int. Cl.: A61F 2/06

(54) **Stent mit einer Stegstruktur**
Stent with a strut structure
Stent avec entretoises

(30) Priorität: 16.03.2004 DE 102004012837
(43) Veröffentlichungstag der Anmeldung: 21.09.2005
(73) Patentinhaber: Admedes Schuessler GmbH, 75179 Pforzheim (DE)
(72) Erfinder: Ehrlinspiel, Michael, 76256 Weingarten (DE); Lange, Alexander, 76135 Karlsruhe (DE); Flaxmeier, Erik, 76307 Karlsbad (DE)
(74) Vertreter: Müller-Boré & Partner Patentanwälte

(56) Entgegenhaltungen:
- EP-A- 1 190 685
- EP-A- 1 236 448
- EP-A- 1 356 789
- US-A1- 2001 029 660
- US-A1- 2002 016 623
- US-B1- 6 520 985

## Beschreibung

Die Erfindung betrifft einen Stent zum Implantieren in einen lebenden Körper, insbesondere einen intraluminalen Stent, mit einer Stegstruktur, von der mindestens zwei Stege an mindestens einem Knotenabschnitt miteinander gekoppelt sind. Ferner betrifft die Erfindung ein Verfahren zum Herstellen eines derartigen Stents.

Stents dieser Art schützen Kanäle lebender Körper, wie z.B. Blutgefäße, Speiseröhre, Harnröhre oder Nierengänge, durch Expandieren ihrer rohrförmigen Stegstruktur im Inneren des Kanals vor Kollabieren oder Verschließen. Sie dienen ferner als Träger von Medikamenten in Körperkanälen und ermöglichen so eine lokale Therapie innerhalb des Kanals.

Die Stegstruktur solcher Stents ist aus einer Vielzahl von Stegen gebildet, die jeweils an Knotenabschnitten miteinander gekoppelt sind und einzelne nebeneinander angeordnete Zellen begrenzen. Durch Aufweiten der einzelnen Zellen kann die Stegstruktur insgesamt expandiert und bei bestimmten Stents nachfolgend durch Verkleinern der Zellen auch wieder verkleinert werden. Die Stege bilden also Verbindungselemente zwischen den Knotenabschnitten, welche im Wesentlichen steif sind und wesentlich zur Stützwirkung eines Stents beitragen.

Damit der Stent an der Kanalwand anliegt, muss er radial im Kanal expandierbar sein. Darüber hinaus muss der Stent im expandierten Zustand seiner Stützfunktion gerecht werden. Ziel ist es daher, den Stent hinsichtlich seines Deformationsverhaltens und der daraus resultierenden Dehnungen und Spannungen optimal auszulegen.

EP 1190685 A offenbart einen Stent mit Wellen oder mäanderförmig angeordneten Stegen, die an Knoten mit mindestens einem anderem Steg des Stents verbunden sind.

EP 1356789 A offenbart einen Stent mit in Längsrichtung verlaufenden gleich langen Stegen, die durch 180°-Bögen miteinander verbunden sind.

Der Erfindung liegt die Aufgabe zugrunde einen Stent mit einer Stegstruktur bereitzustellen, der ein möglichst großes Durchmesserverhältnis zwischen expandiertem und komprimiertem Zustand ermöglicht. Auf diese Weise soll vorteilhaft sowohl der Zugang in sehr kleine Gefäße, als auch das Abstützen sehr großer Gefäße möglich werden.

Die Aufgabe ist erfindungsgemäß mit einem Stent gemäß Anspruch 1 gelöst, bei dem an mindestens einem der Stege nahe dem Knotenabschnitt das Widerstandsmoment des Steges über dessen Längserstreckung hinweg variiert und damit die bei einer Verformung des Stents am Knotenabschnitt auftretenden Spannungen in Längsrichtung des Steges verteilt sind. Ferner wird die Aufgabe erfindungsgemäß durch ein Verfahren gemäß Anspruch 6 gelöst. Vorteilhafte Weiterbildungen eines erfindungsgemäßen Stents sowie Verfahrens sind in den abhängigen Ansprüchen beschrieben.

Bei bekannten Stents kann zwar grundsätzlich ebenfalls ein vergleichsweise großes Größenverhältnis zwischen nicht expandiertem und expandiertem Zustand gewählt werden, die Stents verlieren aber im expandierten Zustand zunehmend an Stützkraft - der so genannten Radialkraft. Die Radialkraft ist aber ein wichtiges Attribut für die Anwendung von Stents.

Die Erfindung basiert auf der Erkenntnis, dass mit zunehmendem Größenverhältnis von Stents sich auch größere Deformationen der Stegstruktur ergeben, die wiederum eine größere Spannung im verwendeten Werkstoff induziert. Überschreiten die Spannungen gegebene Werkstoffgrenzen, im vorliegenden Fall sind dies in der Regel Bruchdehnung bzw. Bruchspannung, dann führt dies zur Beschädigung des jeweiligen Elements innerhalb der Stegstruktur des Stents. Zusätzlich unterliegt das deformierte Element im Anwendungsfall einer wechselnden Dauerbelastung, die bei Überschreiten des werkstoffspezifischen Maximums eine vorzeitige Ermüdung des Bauteils verursacht.

Um dies zu vermeiden, sind bei dem erfindungsgemäßen Stent die induzierten Spannungen selbst bei vergleichsweise hoher Verformungsrate gering und die nach der Verformung, beispielsweise nach einer Expansion erreichbare Festigkeit der Stegstruktur ist vergleichsweise groß. Die erfindungsgemäße Stegstruktur kann daher eine relativ große Anzahl an Wechsel-Verformungen aushalten.

Erfindungsgemäß werden die maximal auftretenden Spannungen verringert, indem die Spannungen gleichmäßig in die Stegstruktur des Stents verteilt werden. Dabei wird die Verformungsenergie von den höchstbelasteten Bereichen in weniger belastete Bereiche verlagert.

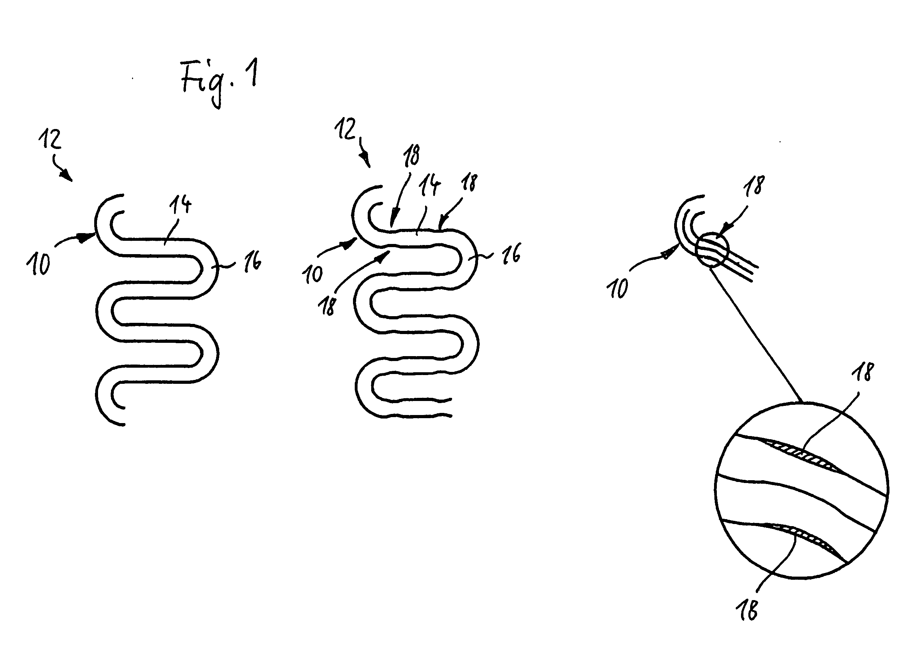

In Fig. 1 ist eine Stegstruktur 10 eines bekannten Stents 12 abschnittsweise dargestellt, an der zur Verteilung von Spannungen innerhalb der Stegstruktur 10 Stege 14 nahe von Knotenabschnitten 16 mit Verjüngungen 18, sogenannten Tapern gestaltet sind. Mit den Tapern soll Verformungsenergie von den höchstbelasteten Bereichen in weniger belastete Bereiche verlagert werden. Da die Struktur an den Tapern auf Grund ihres verringerten Querschnitts mehr Verformungsenergie aufnimmt, entlastet sie die Innenseiten der Biegestellen, die ohne Taper die am stärksten belasteten Stellen sind. Allerdings ist durch die Verjüngungen 18 die Stegstruktur insgesamt geschwächt.

Die durch ein Biegemoment erzeugte maximale Spannung im Stent ist bei gegebenem externen Biegemoment abhängig vom Widerstandsmoment des Bauteils im entsprechenden Querschnitt. Daraus resultiert, dass eine gezielte Beeinflussung des Widerstandsmoments eines Stents nahe von dessen Knotenabschnitten die entstehenden maximalen Spannungen gezielt beeinflussen kann. Das Prinzip des Tapers beeinflusst die maximale Spannung in der Randfaser durch eine tatsächliche Verjüngung eines Steges. Ein verjüngter Steg wirkt sich aber bei der Belastung eines Stents nachteilig aus, weil die Struktur durch die plastische Deformation im verjüngten Bereich stark beansprucht wird. Die Verformungsenergie konzentriert sich dann auf ein relativ geringes Materialvolumen.

Bei wechselnder Biegebelastung eines verjüngten Steges an einem expandierten Stent, welche durch die üblichen Kontraktionen in einer Blutader bedingt ist, wird der verjüngte Bereich zusätzlich zu einer verbleibenden Grundspannung mit einer Wechselspannung belastet. Je geringer die eingeprägte Grundspannung durch die plastische Verformung ist, desto höher kann die überlagerte Wechselspannung sein.

Demgegenüber optimiert die Erfindung die Stegstruktur hinsichtlich verringerter induzierter Spannungen und hinsichtlich der erreichbaren Festigkeit nach einer Verformung der Stegstruktur. Das erfindungsgemäße Wirkprinzip führt dazu, dass die auftretenden Spannungen in den verformten Bereichen nicht in ihrer Gesamtheit verringert, sondern gezielt in weitere Strukturbereiche verteilt werden. Dazu wird erfindungsgemäß gezielt das Widerstandsmoment der verformten Struktur beeinflusst.

Um die erfindungsgemäße Variation des Widerstandsmomentes zu erzielen, ist vorteilhaft der mindestens eine Steg nahe dem Knotenabschnitt über seine Längserstreckung hinweg mit verschieden großen Querschnittsflächen quer zur Längsachse des Steges gestaltet. Mit der Längsachse des Steges ist in diesem Zusammenhang die Achse des Steges gemeint, welche sich im Wesentlichen von einem Knotenabschnitt an einem Ende des Steges zum Knotenabschnitt am entgegen gesetzten Ende des Steges erstreckt. Die zugehörige Querschnittsfläche kann auch als Projektion des Querschnittes auf den Hebelarm des angreifenden Biegemomentes bezeichnet werden. Die Projektion wird vorteilhaft erfindungsgemäß derart variiert, dass das Widerstandsmoment etwas weiter weg von den Innenseiten von Kurven des Steges größer ist als in diesen selbst. Dadurch wird wiederum Verformungsenergie aus der am stärksten belasteten Stelle zumindest teilweise in den Steg hinein verlagert.

Ferner ist gemäß einer bevorzugten Ausführungsform der mindestens eine Steg nahe dem Knotenabschnitt über seine Längserstreckung hinweg mit im Wesentlichen gleichen Querschnittsflächen quer zu seiner Hauptlinie gestaltet. Als Hauptlinie wird in diesem Zusammenhang jene (gedachte) Linie verstanden, entlang der sich der betrachtete Steg erstreckt. Diese Linie ist insbesondere dann gekrümmt, wenn der Steg selbst gekrümmt bzw. gebogen ist. Indem die Querschnittsfläche des erfindungsgemäßen Steges quer zu dieser Hauptlinie erfindungsgemäß stets vergleichsweise groß ist, ist eine Schwächung durch Verjüngungen, wie sie im Stand der Technik auftreten kann, vermieden.

Besonders vorteilhaft ist in der Mantelfläche des Stents betrachtet die Breite des mindestens einen erfindungsgemäßen Steges zumindest nahe dem Knotenabschnitt über dessen Längserstreckung hinweg im Wesentlichen gleich groß. Auf diese Weise ist im Vergleich zu einem verjüngten Steg das Volumen, welches plastische Deformationsenergie aufnimmt, größer. Dadurch ist die nach der plastischen Deformation verbleibende Grundspannung geringer, indem die gleiche Energiemenge auf ein größeres Volumen verteilt wird. Die aufnehmbare Wechselbelastung ist um diesen Anteil größer.

Die genannten Vorteile treten besonders ausgeprägt bei einem erfindungsgemäßen Stent auf, bei dem der mindestens eine Steg nahe dem Knotenabschnitt über seine Längserstreckung hinweg mit gewellter Form gestaltet ist. Die auftretenden Spannungen werden mit dieser Form gezielt in weitere Bereiche der Stegstruktur verteilt, ohne dass die Spannungen in den verformten Bereichen in ihrer Gesamtheit verringert werden.

Besonders vorteilhaft ist der erfindungsgemäße Stent insgesamt mit einer Stegstruktur versehen, bei der jeder einzelne Steg über seine gesamte sich zwischen zwei Knotenabschnitten erstreckende Länge mit gewellter Form gestaltet ist.

Die gewellte Form des mindestens einen Steges kann durch einen Stanz- oder Laserschweißprozess einfach hergestellt werden, indem sie in der Mantelfläche des Stents ausgebildet bzw. ausgeschnitten wird.

Damit die angestrebte Spannungsverteilung im Belastungsfall besonders gleichmäßig wird, sollte die gewellte Form des mindestens einen Steges ferner mit alternierenden Krümmungen mit insbesondere im Wesentlichen gleichen Krümmungsradien gestaltet sein.

Gemäß der Erfindung wird weiterhin ein Verfahren zum Herstellen eines Stents bereitgestellt nach Anspruch 6.

Gemäß einer bevorzugten Ausführungsform der Erfindung wird in dem Ausbildungsschritt der mindestens eine Steg nahe dem Knotenabschnitt über seine Längserstreckung hinweg mit verschieden großen Querschnittsflächen quer zur Längsachse des Steges gestaltet.

Bevorzugt wird in dem Ausbildungsschritt der mindestens eine Steg nahe dem Knotenabschnitt über seine Längserstreckung hinweg mit im Wesentlichen gleichen Querschnittsflächen quer zu seiner Hauptlinie gestaltet.

Weiterhin bevorzugt wird in dem Ausbildungsschritt in der Mantelfläche des Stents betrachtet die Breite des mindestens einen Steges zumindest nahe dem Knotenabschnitt über dessen Längserstreckung hinweg im Wesentlichen gleich groß ausgebildet.

Am Bevorzugtesten wird in dem Ausbildungsschritt der mindestens eine Steg nahe dem Knotenabschnitt über seine Längserstreckung hinweg mit gewellter Form ausgestaltet.

Weiterhin bevorzugt wird in dem Ausbildungsschritt der mindestens eine Steg über seine gesamte sich zwischen zwei Knotenabschnitten erstreckende Länge mit gewellter Form ausgestaltet.

Bevorzugt wird die gewellte Form des mindestens einen Steges in der Mantelfläche des Stents ausgebildet.

Am bevorzugtesten wird die gewellte Form des mindestens einen Steges mit alternierenden Krümmungen mit insbesondere im Wesentlichen gleichen Krümmungsradien ausgestaltet.

Nachfolgend wird ein Ausführungsbeispiel eines erfindungsgemäßen Stents anhand der beigefügten schematischen Zeichnungen näher erläutert. Es zeigt:
- Fig. 1: stark vergrößerte Teilansichten von Stegstrukturen bekannter Stents,
- Fig. 2: eine vergrößerte Draufsicht mit einer Detailvergrößerung der Stegstruktur eines erfindungsgemäßen Stents und
- Fig. 3: eine vergrößerte Draufsicht eines einzelnen Steges der Stegstruktur gemäß Fig. 2 mit simulierten Spannungsbereichen.

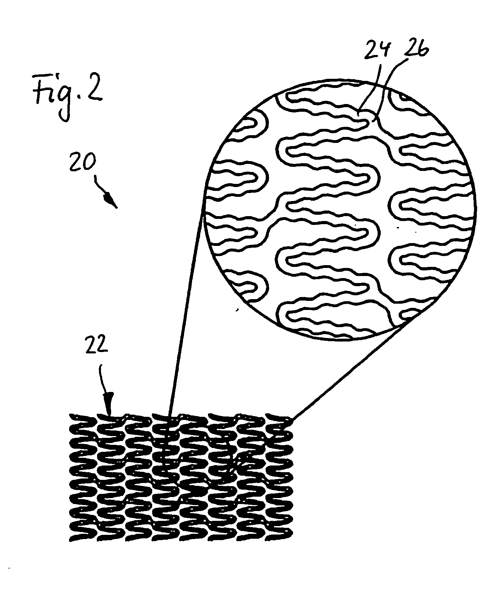

Fig. 2 zeigt im Gegensatz zu der bereits oben erläuterten Fig. 1 einen erfindungsgemäßen Stent 20 mit einer Stegstruktur 22, bei der Stege 24 an Knotenabschnitten 26 miteinander gekoppelt sind.

Die Stege 24 sind über ihre gesamte sich zwischen zwei Knotenabschnitten 26 erstreckende Länge mit einer gewellten Form gestaltet, die aus einem dünnwandigen Material einer Mantelfläche des Stents 20 durch einen Laserschneidprozess ausgeschnitten worden ist. Die gewellte Form ist mit einer Anzahl von Ausbuchtungen als Abfolge konkaver und konvexer Bögen bzw. alternierender Radienelemente gestaltet. Im dargestellten Ausführungsbeispiel sind zirka 7 bis 8 solcher konkaver und konvexer Bögen aufeinanderfolgend an einem einzelnen Steg 24 ausgebildet. Als vorteilhaft sind zwischen 3 und 12, insbesondere zwischen 5 und 10 Bögen ermittelt worden. Die gewellte Form der Stege 24 kann in dem Stent 20 bereichsweise vorgesehen sein, d.h. nicht sämtliche Stege 24 sind mit einer Wellenform versehen und/oder die Wellenform ist lediglich an einem Bereich des einzelnen Stegs 24 vorgesehen. So können die Stege 24 bevorzugt in jenen Bereichen eine Verteilung der während der Ausweitung des Stents 20 auftretenden Spannungen stattfinden, die am stärksten bei der Ausweitung des Stents 20 beansprucht werden.

Alternativ oder zusätzlich kann der Stent durch ein Stanzverfahren hergestellt sein. Der Stent 20 ist dabei vorteilhaft aus rostfreiem Edelstahl oder Kobalt-Chrom-Tantal-Legierungen hergestellt. Der Stent 20 wird bevorzugt durch eine Aufweitungseinrichtung, wie z.B. einen Ballonkatheter im Körper aufgeweitet. Als Materialien eignen sich allgemein vorteilhaft Tantal, Niob oder Kobaltlegierungen. Denkbar sind aber auch Stents aus anderen Werkstoffen, wie z.B. Polymeren, selbstabbaubaren Werkstoffen (z.B. Milchsäure-Werkstoffen bzw. -Derivaten), sowie Stents aus anderen selbstexpandierbaren Werkstoffen bzw. (bevorzugt temperaturabhängigen) Formgedächniswerkstoffen.

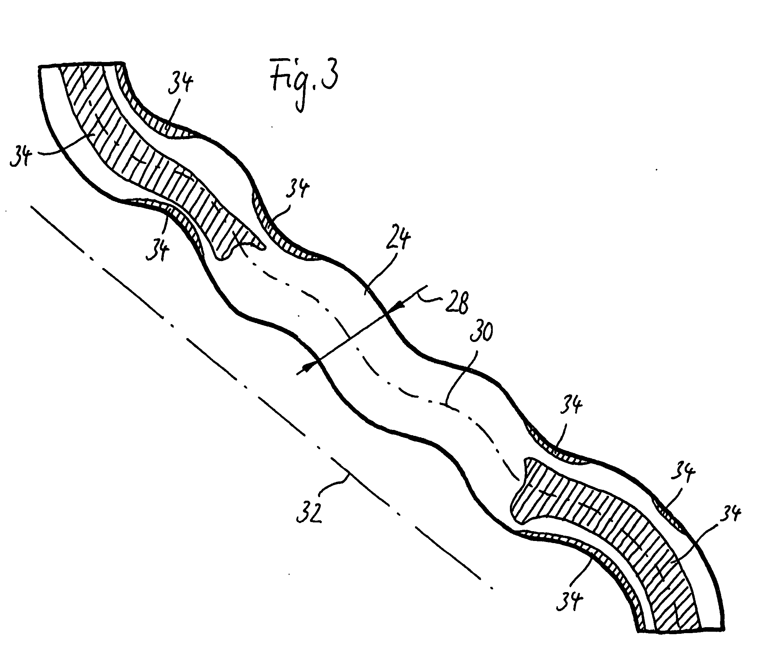

Wie insbesondere in Fig. 3 zu sehen ist, ist in der Mantelfläche des Stents 20 betrachtet die Breite 28 eines jeden Steges 24 über dessen Längserstreckung hinweg im Wesentlichen gleich groß.

Weil ferner auch die Dicke des Materials der Mantelfläche des Stents 20 insgesamt im Wesentlichen gleich ist, ist jeder Steg 24 über seine Längserstreckung hinweg mit quer zu seiner Hauptlinie 30 im Wesentlichen gleichen Querschnittsflächen gestaltet. Über die Längserstreckung des Steges 24 betrachtet sind dabei hingegen die Querschnittsflächen quer zur Längsachse 32 des Steges 24 verschieden groß gestaltet.

Auf diese Weise werden an jedem Steg 24 insbesondere nahe dem zugehörigen Knotenabschnitt 26 die Widerstandsmomente des Steges 24 über dessen Längserstreckung hinweg variiert und die bei einer Verformung des Stents 20 am Knotenabschnitt 26 auftretenden Spannungen in Längsrichtung des Steges zumindest teilweise bzw. bereichsweise verteilt. Spannungen nahe dem Knotenabschnitt 26 sind dadurch in ihrer Gesamtheit zwar nicht verringert, sie werden aber gezielt in weitere Strukturbereiche der Stegstruktur 22 verteilt, was an dem in Fig. 3 vereinfacht dargestellten Bereichen erhöhter Spannung 34 zu erkennen ist.

Die Herstellung des Stents gemäß der Erfindung oder eine bevorzugte Ausführungsform hiervon kann sowohl aus Rohrmaterial als auch aus Flachmaterial erfolgen, wobei bei letzterem der Stent später gerollt, geschweißt und/oder endbearbeitet wird. Weiterhin kann die Herstellung des Stents mittels Laserschneidens, Laserabtrags, photochemischen Ätzens und/oder Erodierens erfolgen. Weiterhin kann die Herstellung des Stents auch derart erfolgen, daß die Stentstruktur in einer zumindest teilweise expandierten Form gefertigt wird und der Stent anschließend zum Einführen z.B. in den Katheter auf eine komprimierte Form verkleinert wird, bevor er nachfolgend im Körper wieder zumindest teilweise expandiert wird.

### Bezugszeichenliste

- 10: Stegstruktur
- 12: Stent
- 14: Steg
- 16: Knotenabschnitt
- 18: Verjüngung
- 20: Stent
- 22: Stegstruktur
- 24: Steg
- 26: Knotenabschnitt
- 28: Breite
- 30: Hauptlinie
- 32: Längsachse
- 34: Bereiche erhöhter Spannung

## Patentansprüche

1. Stent (20) zum Implantieren in einen lebenden Körper, insbesondere intraluminaler Stent, mit einer Stegstruktur (22), von der mindestens zwei Stege (24) an mindestens einem Knotenabschnitt (26) miteinander gekoppelt sind, wobei die Stege (24) sich jeweils zwischen zwei Knotenabschnitten (26) erstrecken, wobei der mindestens eine Steg (24) zwischen zwei Knotenabschnitten (26) mit einer Vielzahl an entlang einer geraden Längsachse (32) verlaufenden Ausbuchtungen als Abfolge konkaver und konvexer Bögen ausgebildet ist, wobei der mindestens eine Steg (24) nahe dem Knotenabschnitt (26) über seine Längserstreckung hinweg mit im Wesentlichen gleichen Querschnittsflächen quer zu seiner Hauptlinie (30) gestaltet ist, so dass an mindestens einem der Stege (24) nahe dem Knotenabschnitt (26) das Widerstandsmoment des Steges (24) über dessen Längserstreckung hinweg variiert und die bei einer Verformung des Stents (20) am Knotenabschnitt (26) auftretenden Spannungen in Längsrichtung des Steges (20) gezielt in weitere Bereiche der Stegstruktur verteilt werden.

2. Stent nach Anspruch 1, wobei
der mindestens eine Steg (24) nahe dem Knotenabschnitt (26) über seine Längserstreckung hinweg mit verschieden großen Querschnittsflächen quer zur Längsachse (32) des Steges (24) gestaltet ist.

3. Stent nach einem der Ansprüche 1 bis 2, wobei
in der Mantelfläche des Stents (20) betrachtet die Breite (28) des mindestens einen Steges (24) zumindest nahe dem Knotenabschnitt (26) über dessen Längserstreckung hinweg im Wesentlichen gleich groß ist.

4. Stent nach einem der vorherigen Ansprüche, wobei
die gewellte Form des mindestens einen Steges (24) in der Mantelfläche des Stents (20) ausgebildet ist.

5. Stent nach einem der vorherigen Ansprüche, wobei
die gewellte Form des mindestens einen Steges (24) mit alternierenden Krümmungen mit insbesondere im Wesentlichen gleichen Krümmungsradien gestaltet ist.

6. Verfahren zum Herstellen eines Stents nach einem der Ansprüche 1 bis 5,
mit den Schritten:
Ausbilden einer Stegstruktur (22) mit mindestens zwei Stegen (24) und einem dazwischen angeordneten Knotenabschnitt (26) zum Koppeln der Stege (24) miteinander, so dass die Stege (24) sich jeweils zwischen zwei Knotenabschnitten (26) erstrecken, und
Ausbilden des Stegs (24) zwischen zwei Knotenabschnitten (26) mit einer Vielzahl an entlang einer geraden Längsachse (32) verlaufenden Ausbuchtungen als Abfolge konkaver und konvexer Bögen, wobei der Steg (24) nahe dem Knotenabschnitt (26) über seine Längserstreckung hinweg mit im Wesentlichen gleichen Querschnittsflächen quer zu seiner Hauptlinie (30) gestaltet ist, so dass dessen Widerstandsmoment über die Längserstreckung des Steges (24) hinweg variiert und die bei einer Verformung des Stents (20) am Knotenabschnitt (26) auftretenden Spannungen in Längsrichtung des Steges (20) gezielt in weitere Bereiche der Stegstruktur werteilt werden.

7. Verfahren nach Anspruch 6, wobei
in dem Ausbildungsschritt der mindestens eine Steg (24) nahe dem Knotenabschnitt (26) über seine Längserstreckung hinweg mit verschieden großen Querschnittsflächen quer zur Längsachse (32) des Steges (24) gestaltet wird.

8. Verfahren nach einem der Ansprüche 6 bis 7, wobei
in dem Ausbildungsschritt in der Mantelfläche des Stents (20) betrachtet die Breite (28) des mindestens einen Steges (24) zumindest nahe dem Knotenabschnitt (26) über dessen Längserstreckung hinweg im Wesentlichen gleich groß ausgebildet wird.

9. Verfahren nach einem der Ansprüche 6 bis 8, wobei
die gewellte Form des mindestens einen Steges (24) in der Mantelfläche des Stents (20) ausgebildet wird.

10. Verfahren nach einem der Ansprüche 6 bis 9, wobei
die gewellte Form des mindestens einen Steges (24) mit alternierenden Krümmungen mit insbesondere im Wesentlichen gleichen Krümmungsradien ausgestaltet wird.

## Claims

1. Stent (20) intended for implantation in a living body, in particular an intraluminal stent, and having a bridge structure (22) in which at least two bridges (24) are coupled to one another at at least one node region (26), wherein the bridges (24) each extend between two node regions (26), wherein the at least one bridge (24) between two node regions (26) is formed with a multiplicity of bulges extending along a straight longitudinal axis (32) as a sequence of concave and convex arcs, wherein the at least one bridge (24) is designed, near the node region (26), along its length, with substantially identical cross-sectional surfaces transverse to its main line (30), such that on at least one of the bridges (24) near the node region (26), the section modulus of the bridge (24) varies along the length thereof, and the stresses arising at the node region (26) upon deformation of the stent (20) are distributed in the longitudinal direction of the bridge (20) into other areas of the bridge structure in a targeted manner.

2. Stent according to Claim 1, wherein the at least one bridge (24) near the node region (26) is designed along its length with different sizes of cross-sectional areas transverse to the longitudinal axis (32) of the bridge (24).

3. Stent according to one of Claims 1 to 2, wherein, viewed in the jacket surface of the stent (20), the width (28) of the at least one bridge (24), at least near the node region (26), is substantially the same size along the length thereof.

4. Stent according to one of the preceding claims, wherein the undulated shape of the at least one bridge (24) is formed in the jacket surface of the stent (20).

5. Stent according to one of the preceding claims, wherein the undulated shape of the at least one bridge (24) is designed with alternating curves with in particular substantially identical radii of curvature.

6. Method for producing a stent according to one of Claims 1 to 5, with the following steps:
forming a bridge structure (22) with at least two bridges (24) and a node region (26) arranged between them for coupling the bridges (24) to one another, such that the bridges (24) each extend between two node regions (26) and
forming the bridge (24) between two node regions (26) with a multiplicity of bulges extending along a straight longitudinal axis (32) as a sequence of concave and convex arcs, wherein the bridge (24) is designed, near the node region (26), along its length, with substantially identical cross-sectional surfaces transverse to its main line (30), in such a way that its section modulus varies along the length of the bridge (24), and the stresses arising at the node region (26) upon deformation of the stent (20) are distributed in the longitudinal direction of the bridge (20) into other areas of the bridge structure in a targeted manner.

7. Method according to Claim 6, wherein, in the formation step, the at least one bridge (24) near the node region (26) is designed along its length with different sizes of cross-sectional areas transverse to the longitudinal axis (32) of the bridge (24).

8. Method according to one of Claims 6 to 7, wherein, in the formation step, viewed in the jacket surface of the stent (20), the width (28) of the at least one bridge (24), at least near the node region (26), is substantially the same size along its length.

9. Method according to one of Claims 6 to 8, wherein the undulated shape of the at least one bridge (24) is formed in the jacket surface of the stent (20).

10. Method according to one of Claims 6 to 9, wherein the undulated shape of the at least one bridge (24) is designed with alternating curves with in particular substantially identical radii of curvature.

## Revendications

1. Stent ou dilatateur (20) pour l'implantation dans un corps vivant, en particulier stent intraluminal, avec une structure d'entretoises (22), dont au moins deux entretoises (24) sont accouplées entre elles sur au moins une section nodale (26), les entretoises (24) s'étendant respectivement entre deux sections nodales (26), dans lequel au moins une entretoise (24) est configurée, entre deux sections d'entretoise (26), avec une pluralité de cambrages s'étendant le long d'un axe longitudinal droit (32) sous la forme d'arcs concaves et convexes, dans lequel est formée au moins une entretoise (24) à proximité du tronçon nodal (26) sur son extension longitudinale d'une surface transversale sensiblement identique transversalement à sa ligne principale (30), de sorte que, sur au moins l'une des entretoises (24) à proximité du tronçon nodal (26), le moment de résistance de l'entretoise (24) sur son extension longitudinale varie et les tensions se produisant lors d'une déformation du stent (20) sur le tronçon nodal (26) sont réparties dans la direction longitudinale de l'entretoise (24) de façon ciblée dans l'autres zones de la structure d'entretoises.

2. Stent selon la revendication 1, dans lequel au moins une entretoise (24) à proximité du tronçon nodal (26) est formée sur son extension longitudinale avec des surfaces transversales de grandeurs différentes, transversalement à l'axe longitudinal (32) de l'entretoise (24).

3. Stent selon l'une des revendications 1 à 2, dans lequel, dans la surface d'enveloppe du stent (20), la largeur (28) de au moins une entretoise (24), au moins à proximité du tronçon nodal (26) est de dimension sensiblement égale sur son extension longitudinale.

4. Stent selon l'une des revendications précédentes, dans lequel la forme ondulée de au moins une entretoise (24) est configurée dans la surface d'enveloppe du stent (20).

5. Stent selon l'une des revendications précédentes, dans lequel la forme ondulée de au moins une entretoise (24) est formée avec des courbures en alternance, avec en particulier des rayons de courbure sensiblement égaux.

6. Procédé pour la fabrication d'un stent selon l'une des revendications 1 à 5, comportant les étapes consistant à :
former une structure d'entretoise (22) avec au moins deux entretoises et un tronçon nodal (26) disposé dans la partie intermédiaire pour l'accouplement des entretoises (24) entre elles, de sorte que les entretoises (24) s'étendent respectivement entre deux tronçons nodaux (26), et
configurer l'entretoise (24) entre deux tronçons d'entretoise (26) avec une pluralité de cambrages s'étendant le long d'un axe longitudinal droit (32) sous la forme d'arcs concaves et convexes, dans lequel l'entretoise (24) à proximité du tronçon nodal (26) est configurée sur son extension longitudinale avec des surfaces transversales sensiblement égales transversalement par rapport à sa ligne principale (30), de sorte que son moment de résistance sur l'extension longitudinale de l'entretoise (24) est variable et les tensions se produisant lors d'une déformation de l'entretoise (20) sur le tronçon nodal (26) sont réparties dans la direction longitudinale de l'entretoise (24) de façon ciblée dans d'autres zones de la structure d'entretoises.

7. Procédé selon la revendication 6, dans lequel, dans l'étape de configuration de au moins une entretoise (24) à proximité du tronçon nodal (26) sur son extension longitudinale, sont configurées des surfaces transversales de grandeurs différentes, transversalement à l'axe longitudinal (32) de l'entretoise (24).

8. Procédé selon l'une des revendications 6 à 7, dans lequel, dans l'étape de configuration dans la surface d'enveloppe du stent (20), la largeur (28) de au moins une entretoise (24) est configurée au moins à proximité du tronçon nodal (26) sur son extension longitudinale avec une dimension sensiblement égale.

9. Procédé selon l'une des revendications 6 à 8, dans lequel la forme ondulée de au moins une entretoise (24) est configurée dans la surface d'enveloppe du stent (20).

10. Procédé selon l'une des revendications 6 à 9, dans lequel la forme ondulée de au moins une entretoise (24) est conçue avec des courbures alternées et avec, en particulier, des rayons de courbure sensiblement égaux.
